# EUROPEAN PATENT APPLICATION

(11) **EP 3 549 527 A1**
(43) Date of publication of application: **09.10.2019**
(21) Application number: 19165799.8
(22) Date of filing: 28.03.2019
(51) Int. Cl.: A61B 8/00

(54) **PROBE RACK AND ULTRASOUND APPARATUS EMPLOYING THE SAME**

(30) Priority: 06.04.2018 KR 20180040609
(71) Applicant: Samsung Medison Co., Ltd., Hongcheon-gun, Gangwon-do, 25108 (KR)
(72) Inventor: HAN, Kiwook, Gyeonggi-do (KR); LEE, Taeho, Gyeonggi-do (KR)
(74) Representative: Hylarides, Paul Jacques

(57) **Abstract**

Provided are a probe rack and an ultrasound apparatus employing the probe rack. The probe rack includes: a probe base on which a probe is placed; a hanger over which a probe cable of the probe is hung; and a movable support configured to movably support the hanger with respect to the probe base.

## Description

### BACKGROUND

### 1. Field

The disclosure relates to probe racks for holding probes and ultrasound apparatuses employing the probe racks.

### 2. Description of Related Art

Ultrasound apparatuses transmit ultrasound signals generated in a body surface of an object to a predetermined internal part of the object and obtain an image of blood flow or a cross-section of a soft tissue by using information of ultrasound signals reflected from an inner tissue of the object. Ultrasound apparatuses are compact, affordable, and display images in real-time. Being highly safe due to lack of radiation exposure, such ultrasound apparatuses have been widely used together with other types of imaging diagnosis apparatuses such as an X-ray diagnostic apparatus, a computed tomography (CT) scanner, a magnetic resonance imaging (MRI) apparatus, a nuclear medicine diagnostic apparatus, etc.

Probes configured to transmit or receive ultrasound signals are generally connected to a main body via cables, and are hung on a probe holder positioned on a side surface of a control panel.

### SUMMARY

Provided are probe racks configured to provide improved user convenience and ultrasound apparatuses employing the probe racks.

Additional aspects will be set forth in part in the description which follows and, in part, will be apparent from the description, or may be learned by practice of the presented embodiments.

In accordance with an aspect of the disclosure, a probe rack includes: a probe base on which a probe is placed; a hanger over which a probe cable of the probe is hung; and a movable support configured to movably support the hanger with respect to the probe base.

The hanger may be located at one side of the probe base.

The hanger may extend in one direction.

The movable support may include an elastic member for applying an elastic force to movement of the hanger or a damper for mitigating collision.

The movable support may enable the hanger to move automatically or manually.

The hanger may be located below the probe base.

The movable support may be formed integrally with or be detachably attached to the probe base.

The probe rack may further include a hanger actuator that is controlled by an electrical signal to move the hanger in a first direction with respect to the probe base.

The hanger may include first and second hangers, and the hanger actuator may be further configured to independently move the first and second hangers. For example, the hanger actuator may include first and second hanger actuators respectively provided for the first and second hangers.

The probe rack may further include a probe sensor configured to detect whether the probe is placed on the probe base.

The probe rack may further include an indicator light configured to emit light according to a result of the detecting by the probe sensor.

The probe rack may further include a cable sensor configured to detect whether the probe cable is hung over the hanger.

The probe rack may further include an indicator light configured to emit light according to a result of the detecting by the cable sensor.

The probe rack may further include a touch sensor located on the hanger or probe base, and when a touch is detected by the touch sensor, the hanger actuator may move the hanger.

The indicator light may be located on the hanger or probe base.

In accordance with another aspect of the disclosure, an ultrasound apparatus includes: a probe configured to transmit and receive ultrasound signals; and a probe rack configured to hold the probe, and the probe rack includes: a probe base on which a probe is placed; a hanger over which a probe cable of the probe is hung; and a movable support configured to movably support the hanger with respect to the probe base.

The ultrasound apparatus may further include a user input interface configured to input a control command according to manipulation by a user, and the probe rack may be spatially separated from the user input interface. In this case, the user input interface may include at least one of a touch screen and a control panel including at least one hardware buttons.

The ultrasound apparatus may further include a probe rack support configured to support the probe rack while enabling the probe rack to move independently of the user input interface.

The ultrasound apparatus may further include a controller configured to control the ultrasound apparatus, and the movable support may include a hanger actuator configured to move the hanger in a first direction with respect to the probe base. When a probe select command is input to the control panel, the controller may control the hanger actuator to move the hanger.

The probe rack may further include a probe sensor configured to detect whether the probe is placed on the probe base and an indicator light configured to emit light according to a result of the detecting by the probe sensor, and the controller may control the indicator light to emit light according to the result of the detecting by the probe sensor.

The controller may include a probe setting mode, and the controller may control, in the preset setting mode, the indicator light to emit light according to the result of the detecting by the probe sensor.

The probe rack may further include a cable sensor configured to detect whether the probe cable is hung over the hanger and an indicator light configured to emit light according to a result of the detecting by the cable sensor. The controller may control the indicator light to emit light according to the result of the detecting by the cable sensor.

The probe rack may further include a touch sensor located on the hanger or probe base, and the controller may control, when a touch is detected by the touch sensor, the hanger actuator to move the hanger.

The ultrasound apparatus may further include a control panel configured to input a control command according to manipulation by a user. The probe base may be attached to a side surface of the control panel, and the hanger may be located below the control panel.

The probe rack may be affixed to the control panel, and the probe base may be a probe holder into which the probe is fit.

The hanger may be attached to or separated from a bottom surface of the control panel.

In accordance with another aspect of the disclosure, an ultrasound apparatus includes: a main body; a probe rack configured to hold a probe; and a user input interface configured to input a control command according to manipulation by a user, and the probe rack is spatially separated from the user input interface such that the main body is interposed therebetween.

The ultrasound apparatus may further include an examinee chair or an examinee table located in a space for accommodating an examinee. The probe rack may be spatially separated from the user input interface such that the examinee chair or the examinee table is interposed therebetween.

The ultrasound apparatus may further include a probe rack support configured to support the probe rack while enabling the probe rack to move independently of the main body.

The probe rack support may be further configured to support the probe rack while enabling the probe rack to move from one side of the main body or the space for accommodating the examinee to an opposite side thereof.

The ultrasound apparatus may further include a user input interface support configured to movably support the user input interface, and the user input interface support may be further configured to support the user input interface while enabling the user input interface to move from one side of the main body or the space for accommodating the examinee to an opposite side thereof.

The ultrasound apparatus may further include a support column erected perpendicular to a main body and first and second coupling members configured to respectively rotatably couple a probe rack support and a user input interface support to the support column.

A length of the user input interface support with respect to a diameter direction of the support column may be greater than that of the probe rack support, and a position where the user input interface support is coupled to the support column via the first coupling member may be located below a position where the probe rack support is coupled to the support column via the second coupling member.

The length of the user input interface support with respect to the diameter direction of the support column may be less than that of the probe rack support, and the position where the user input interface support is coupled to the support column via the first coupling member may be located above the position where the probe rack support is coupled to the support column via the second coupling member.

The probe rack support may support the probe rack while enabling it to move up and down.

The probe rack support may tiltably support the probe rack.

The ultrasound apparatus may further include a rack actuator that is controlled by an electrical signal to move the probe rack.

The ultrasound apparatus may be configured to enable the probe rack to move toward the user according to manipulation by the user.

The ultrasound apparatus may further include a foot switch or lever for manipulating driving of the rack actuator.

A switch for manipulating driving of the rack actuator may be provided on the control panel. The switch may be a software or hardware switch.

### BRIEF DESCRIPTION OF THE DRAWINGS

The above and other aspects, features, and advantages of certain embodiments of the present disclosure will be more apparent from the following description taken in conjunction with the accompanying drawings, in which:
FIG. 1 is an external view of an ultrasound apparatus according to an embodiment;
FIG. 2 is a block diagram of a configuration of an ultrasound apparatus according to an embodiment;
FIG. 3 is an external view of a probe rack according to an embodiment;
FIG. 4 illustrates a configuration of a probe rack according to an embodiment;
FIG. 5 is a flowchart illustrating an operation of an ultrasound apparatus according to an embodiment;
FIG. 6 illustrates a user input interface of an ultrasound apparatus related to an embodiment;
FIG. 7 illustrates an example of a probe setting start screen of an ultrasound apparatus related to an embodiment;
FIG. 8 illustrates a configuration of a probe rack according to an embodiment;
FIG. 9 illustrates a configuration of a probe rack according to an embodiment;
FIG. 10 illustrates a configuration of a probe rack according to an embodiment;
FIG. 11 is an external view of an ultrasound apparatus according to an embodiment;
FIG. 12 is an external view of an ultrasound apparatus according to an embodiment;
FIGS. 13 and 14 are diagrams illustrating operations of an ultrasound apparatus according to an embodiment;
FIG. 15 is a diagram illustrating an operation of an ultrasound apparatus according to an embodiment;
FIG. 16 is an external view of an ultrasound apparatus according to an embodiment;
FIG. 17 is an external view of an ultrasound apparatus according to an embodiment; and
FIGS. 18 and 19 are diagrams illustrating a probe rack according to an embodiment.

### DETAILED DESCRIPTION

The terms used in the specification are general terms currently widely used in the art based on functions described in embodiments, but may have different meanings according to an intention of one of ordinary skill in the art, precedent cases, or advent of new technologies. Also, some terms may be arbitrarily selected by the applicant, and in this case, the meaning of the selected terms will be described in detail in the detailed description of the disclosure. Thus, the terms used herein have to be defined not as simple names but based on the meaning of the terms together with the overall description of the disclosure.

Throughout the specification, it will also be understood that when a part "includes" or "consists of' an element, unless there is a particular description contrary thereto, the part may further include other elements, not excluding the other elements. Furthermore, terms such as "... unit", "... module", and the like refer to units that perform at least one function or operation, and the unit or the module may be implemented as hardware, software or a combination of hardware and software. Expressions such as "at least one of," when preceding a list of elements, modify the entire list of elements and do not modify the individual elements of the list.

Throughout the specification, "an ultrasound image" refers to an image of an object, which is obtained using ultrasound waves. The object may refer to a part of the body. For example, the object may include an organ such as the liver, heart, uterus, brain, breast, or abdomen, or a fetus.

Furthermore, an ultrasound image may take different forms. For example, an ultrasound image may be at least one of a brightness (B) mode image, a color (C) mode image, and a Doppler (D) mode image. In addition, according to an embodiment, an ultrasound image may be a two-dimensional (2D) or three-dimensional (3D) image.

Furthermore, throughout the specification, a "user" may be, but is not limited to, a medical expert, such as a medical doctor, a nurse, a medical laboratory technologist, or a medical imaging specialist.

Embodiments will be described more fully hereinafter with reference to the accompanying drawings. In the drawings, like reference numerals denote the same or corresponding elements, and repeated descriptions thereof will be omitted.

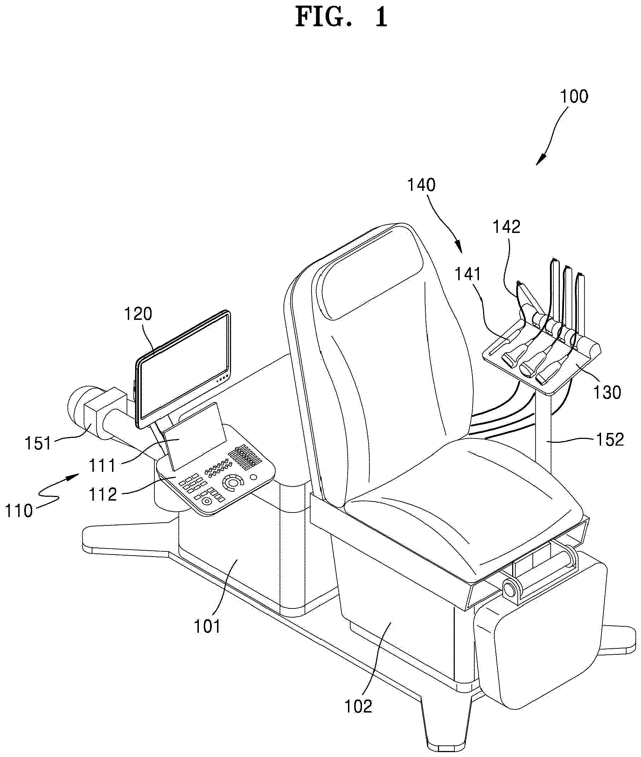

FIG. 1 is an external view of an ultrasound apparatus 100 according to an embodiment.

Referring to FIG. 1, the ultrasound apparatus 100 may include a user input interface 110, a display 120, and a probe rack 130.

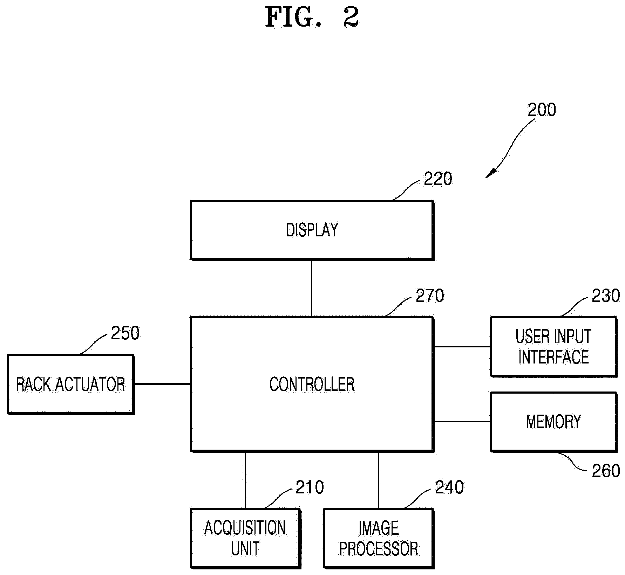

The user input interface 110, the display 120, and the probe rack 130 may each be connected to and supported by a main body 101. A power supply (not shown), an image processor (240 of FIG. 2), and a controller (270 of FIG. 2) may be mounted on the main body 101. The main body 101 may assume an external appearance that it is separate from the user input interface 110 and the display 120, but is not limited thereto. For example, the main body 101 may be externally integrated with the user input interface 110 or the display 120.

According to an embodiment, the ultrasound apparatus 100 may further include an examinee chair 102 on which an examinee is to be seated. The examinee chair 102 may a reclining chair or a chair with an adjustable height. The examinee chair 102 is merely an example, and the ultrasound apparatus 100 may include an examinee table on which the examinee lies during an ultrasound examination. Alternatively, a space where the examinee chair 102 is located may be empty, so the examinee may stand in the empty space. While FIG. 1 shows that the examinee chair 102 is located at one side of the main body 101, the examinee chair 102 may be located on the main body 101.

According to an embodiment, the user input interface 110 may include a touch screen 111 and a control panel 112. The control panel 112 may be movably coupled to the main body 101. For example, as shown in FIG. 1, the control panel 112 may be supported by a user input interface support 151 having one or more joints. The touch screen 111 may be tiltably attached to a top plate of the control panel 112 such that a user may adjust his or her viewing angle. As another example, the touch screen 111 may be slidably affixed to the top plate of the control panel 112 such that its distance from the user may be adjusted. As another example, the touch screen 111 may be movably attached to a position that is not the control panel 112 (e.g., the main body 101). As another example, the touch screen 111 may be removably attached to the control panel 112 or the main body 101.

According to an embodiment, the touch screen 111 may display an ultrasound image, a control menu, etc. Furthermore, the touch screen 111 may display a probe list including identification (ID) information of a probe 140 connected to the ultrasound apparatus 100, a plurality of parameter values set by the user, a preset item list predetermined by a system or the user, etc.

According to an embodiment, in a probe preset mode, a probe setting start screen may be displayed on the touch screen 111, and a soft button corresponding to a probe list is activated, thereby allowing the user to select the desired probe 140.

According to an embodiment, the control panel 112 may include a plurality of hardware buttons for controlling the ultrasound apparatus 100, but embodiments are not limited thereto. Hardware buttons on the control panel 112 may include, but are not limited to, a key pad, a dome switch, a touch pad (a capacitive overlay type, a resistive overlay type, an infrared beam type, a surface acoustic wave type, an integral strain gauge type, a piezoelectric type, etc.), a jog wheel, a jog switch, etc.

According to an embodiment, a probe select button on the control panel 112 may allow the user to select the desired probe 140.

Although it has been described that the user input interface 110 may include both the touch screen 111 and the control panel 112, embodiments are not limited thereto. The user input interface 110 may be composed of only the touch screen 111 or only the control panel 112. Alternatively, the user input interface 110 may include the touch screen 111 at the center and one or more hardware buttons arranged irregularly, instead of_a control panel form in which hardware buttons are grouped together.

The display 120 may be movably coupled to the main body 101. For example, as shown in FIG. 1, the display 120 may be supported together with the control panel 112 via the user input interface support 151. As another example, the display 120 may be coupled to the main body 101 via a separate support member.

The display 120 may display an ultrasound image of an object. For example, the display 120 may display a B mode image, a C mode image, a D mode image, a 2D image, or a 3D image.

The display 120 may include at least one of a liquid crystal display (LCD), a thin film transistor-liquid crystal display (TFT-LCD), an organic light-emitting diode (OLED), a flexible display, a 3D display, and an electrophoretic display.

Although it has been described that the display 120 is provided separately from the touch screen 111 of the user input interface 110, embodiments are not limited thereto. As another example, the touch screen 111 may perform functions of the display 120, and the ultrasound apparatus 100 may not include the display 120. As another example, the ultrasound apparatus 100 may include two or more displays 120 according to its implemented configuration.

According to an embodiment, an ultrasound image or a control menu displayed on the touch screen 111 may be displayed on the display 120 as well. Alternatively, only an ultrasound image selected among a plurality of ultrasound images displayed on the touch screen 111 may be displayed on the display 120.

The probe rack 130 accommodates a plurality of probes 140.

According to an embodiment, the probe rack 130 may be spatially separate from the user input interface 110 (e.g., the control panel 112). Furthermore, the probe rack 130 may be separated from the user input interface 110 such that the main body 101 or the examinee chair 102 is located therebetween.

According to an embodiment, the probe rack 130 may be movably supported by a probe rack support 152 extending from the main body 101. For example, the probe rack support 152 may support the probe rack 130 while enabling it to rotate to the left or right relative to a vertical axis direction. As another example, the probe rack support 152 may support the probe rack 13 to be horizontally movable such that a distance between the probe rack 130 and the user may be adjusted. As another example, the probe rack support 152 may support the probe rack 130 to be vertically movable such that a height of the probe rack 130 may be adjusted. As another example, the probe rack support 152 may be configured to enable two or more complex movements of the probe rack 130 from among left-right rotation, horizontal movement, and vertical movement. The probe rack support 152 may include a joint, a hinge, a lift, etc. Furthermore, the ultrasound apparatus 100 may further include a rack actuator (not shown) configured to move the probe rack support 152 such that the probe rack 130 may move in response to an electrical signal. For example, the ultrasound apparatus 100 may have a probe setting mode, and when the probe setting mode is activated, the probe rack 130 may move toward the user automatically or according to the user's selection.

The probes 140 come in various types according to the purpose of use. According to an embodiment, the probes 140 may include at least one of a cardiac probe, a probe for blood vessel and micro-tissue, an abdominal probe, and an obstetrics/genecology (OB/GYN) probe. As another example, the probes 140 may be of many different types according to a driving method and an external appearance. For example, the probes 140 may include at least one of a 1D probe, a 1.5D probe, a 2D (matrix) probe, and a 3D probe.

Each of the probes 140 (hereinafter called 'the probe 140') includes a probe body 141 and a cable 142. The probe body 141 includes an ultrasound transducer for transmitting and receiving ultrasound signals. The probe body 141 has various shapes depending on the purpose of use, a driving method, etc. The cable 142 electrically connects the probe body 141 to a probe connection terminal (not shown) of the ultrasound apparatus 100.

The ultrasound apparatus 100 may check ID information of the probe 140 connected to the probe connection terminal. For example, the ultrasound apparatus 100 may receive or read ID information prestored in the probe 140. In addition, one or a plurality of probe connection terminals for connecting the cable 142 of the probe 140 may be provided in the main body 101, but embodiments are not limited thereto. As another example, the probe connection terminal may be provided on the examinee chair 102 or the control panel 112.

Since an ultrasound apparatus of the related art has a probe rack positioned on a control panel side, the examinee may often come into contact with unused probes during preparation or actual use. Furthermore, in the ultrasound apparatus of the related art, a cable of a probe is located in a space among a control panel, a user, and an examinee, which may cause inconvenience to the user while using the probe.

On the other hand, the ultrasound apparatus 100 according to the present embodiment includes the probe rack 130 spatially separated from the user input interface 110 (e.g., the control panel 112). This configuration may not only prevent the examinee from often coming into contact with an unused probe during preparation or actual use, but also reduce inconvenience to the user that may be caused by the cable 142 of the probe 140 while using the probe 140.

FIG. 2 is a block diagram of a configuration of an ultrasound apparatus 200 according to an embodiment. Referring to FIG. 2, the ultrasound apparatus 200 according to the embodiment may include an acquisition unit 210, a display 220, a user input interface 230, an image processor 240, a rack driver 250, a memory 260, and a controller 270. However, all of the components shown in FIG. 2 are not essential components. The ultrasound apparatus 200 may include more or fewer components than those shown in FIG. 2.

The acquisition unit 210 may acquire ultrasound data with respect to an object. According to an embodiment, the ultrasound data may be 2D or 3D ultrasound data with respect to the object.

According to an embodiment, the acquisition unit 210 may include the probe (140 of FIG. 1) for transmitting or receiving an ultrasound signal and a beamformer (not shown) for performing transmit focusing and receive focusing on an ultrasound signal.

For example, the display 220 may display and output information processed by the ultrasound apparatus 200. For example, the display 220 may display an ultrasound image of the object, and may also display a user interface (UI) or a graphic UI (GUI) related to function setting.

When the display 220 is formed as a touch screen including a layered structure of a display panel and a touch pad, the display 220 may be used as an input device as well as an output device.

The user input interface 230 refers to an input device via which the user inputs data for controlling the ultrasound apparatus 200. For example, the user input interface 230 may be, but is not limited to, a key pad, a dome switch, a touch pad (a capacitive overlay type, a resistive overlay type, an infrared beam type, a surface acoustic wave type, an integral strain gauge type, a piezoelectric type, etc.), a jog wheel, or a jog switch. In particular, when a touch pad and a display panel form a layered structure as described above, the structure may be referred to as a touch screen.

The image processor 240 may process ultrasound data acquired by the acquisition unit 210 to generate ultrasound image data. The generated ultrasound image data may be transmitted to the display 220 and then displayed as an ultrasound image.

According to an embodiment, when the probe rack (130 of FIG 1) includes a rack actuator, the rack driver 250 may drive the rack actuator to control movement of a hanger in the probe rack 130. As another example, when the probe rack 130 includes an indicator light, the rack driver 250 may control the indicator light provided on the side of a hanger where the probe 140 selected via the user input interface 230 is placed to emit light. As another example, when the probe rack 130 includes a sensor for sensing the probe body (141 of FIG. 1) or the cable (142 of FIG. 1), the rack driver 250 may display the sensed probe 140 on the display 220 or the user input interface 230.

The memory 260 may store programs for processing and control by the controller 270, and store input and output data (e.g., an ultrasound image, examinee information, probe information, probe rack information, application information, a body marker, etc.).

The probe information may contain ID information of a probe, its usage history, etc. The probe rack information may include information about a probe currently being held in a probe rack, position information of the probe rack, etc.

The memory 260 may be an internal or external storage medium. For example, the memory 260 may include at least one storage medium from among a flash memory-type storage medium, a hard disk-type storage medium, a multimedia card micro-type storage medium, card-type memories (e.g., an SD card, an XD memory, and the like), a Random Access Memory (RAM), a Static Random Access Memory (SRAM), a Read-Only Memory (ROM), an Electrically Erasable Programmable ROM (EEPROM), a PROM, a magnetic memory, a magnetic disc, and an optical disc. Furthermore, the ultrasonic apparatus 200 may utilize a web storage or a cloud server that functions as the memory 260 online.

The controller 270 controls all operations of the ultrasound apparatus 200. In other words, the controller 270 may control operations of the acquisition unit 210, the display 220, the user input interface 230, the image processor 240, the rack driver 250, and the memory 260.

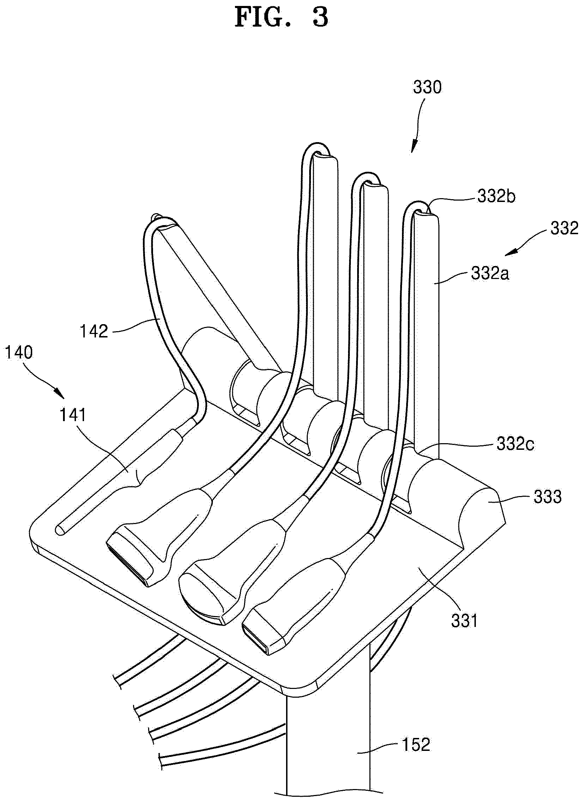

FIG. 3 is an external view of a probe rack 330 according to an embodiment.

Referring to FIG. 3, the probe rack 330 may include a probe base 331, a hanger 332, and a movable support 333. Like in the ultrasound apparatus 100 of FIG. 1, the probe rack 330 may be spatially separated from a user input interface.

Probe bodies 141 are placed on the probe base 331. According to an embodiment, the probe base 331 may have a form of a shelf, but is not limited thereto. As another example, the probe base 331 may have a form of a holder.

The hanger 332 may be located at one side of the probe base 331. The probe rack 330 may include a plurality of hangers, but embodiments are not limited thereto.

According to an embodiment, the hanger 332 includes a hanging section on which the cable 142 of the probe 140 is hung. For example, the hanger 332 may include a rod 332a that extends in one direction, a hanging section 332b located at one end of the rod 332a and on which the cable 142 is hung, and a connector 332c that is located at the other end of the rod 332a and rotatably connected to the movable support 333. The rod 332a may be formed to have a semicylindrical shape in which the cable 142 is placed, but is not limited thereto. As another example, the rod 332a may have a form of column. The hanging section 332b may be the end of the rod 332a itself, but is not limited thereto. As another example, the hanging section 332b may be formed by making the end of the rod 332a a little smaller and rounder, thereby slightly resisting separation of the cable 142 from the hanger 332. As another example, the hanging section 332b may be formed in the shape of a hook at a portion that is not the end of the rod 332a.

The movable support 333 may support the hanger 332 such that the hanger 332 may manually move with respect to the probe base 331. According to an embodiment, as shown in FIG. 3, the movable support 333 may rotate the hanger 332 by attaching the connector 332c of the hanger 332 to a rotation axis, but is not limited thereto. As another example, the movable support 333 may extend or stretch out the hanger 332 in a longitudinal direction. As another example, the movable support 333 may rotate the hanger 332 while extending/stretching out the same. In addition, an elastic member, a shock absorber, a damper, or the like may be added to the movable support 333 such that the hanger 332 may support the weight of the cable 142 or may move smoothly.

The movable support 333 may be formed integrally with or be detachably attached to the probe base 331.

The cable 142 of the probe 140 is hung on the hanging section 332 of the hanger 332 while the hanger 332 is vertically erected. When the user holds the probe 140 in his or her hand and pulls it, the hanger 332 rotates such that its end faces the user, and the cable 142 moves toward the user and is then separated from the hanger 332 so that the probe 140 is ready for use.

The cable 142 of the probe 140 is thick and heavy because it contains a number of wires for inputting or outputting electrical signals corresponding to ultrasound signals. According to the related art, since the cable 142 is draped down toward the floor, the user has to pull the cable 142 upward in order to use the probe 140. Thus, the user feels the weight of the cable 142 and is inconvenienced by having to pull it up. Furthermore, in the related art, when the plurality of probes 140 are respectively connected to probe connection terminals of the ultrasound apparatus 100, cables may be entangled with one another while being draped down toward the floor. On the other hand, in the ultrasound apparatus 100 according to the present embodiment, the hanger 332 is configured to hang the cable 142 from a side close to the probe body 141, thereby sharing a part of the weight of the cable 142. Furthermore, as the hanger 332 moves, the cable 142 naturally comes out of the hanger 332 when the user pulls the probe 140 for use. Thus, in the ultrasound apparatus 100 according to the embodiment, the user may feel less weight of the cable 142 when pulling the probe 140 for use, so user convenience may be improved. Furthermore, even when the plurality of probes 140 are respectively connected to probe connection terminals of the ultrasound apparatus 100, it is possible to prevent their corresponding cables 142 from being entangled with one another by hanging the cables 142 in the hanger 332.

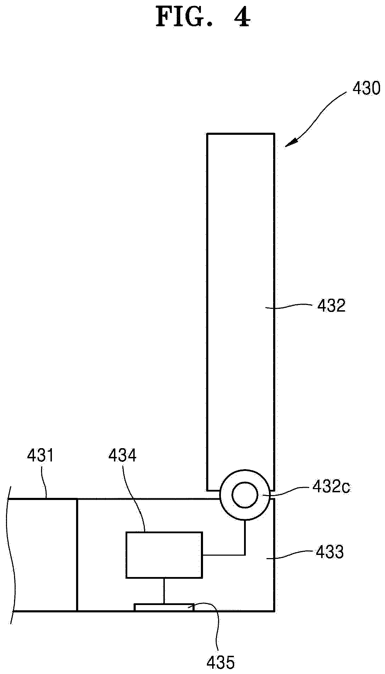

FIG. 4 illustrates a configuration of a probe rack 430 according to an embodiment.

Referring to FIG. 4, the probe rack 430 according to the present embodiment may include a probe base 431, the hanger 432, and a movable support 433. Since the probe rack 430 may have substantially the same configuration as the probe rack 330 described with reference to FIG. 3 except that the probe rack 430 further includes a hanger actuator 434, only the difference is described, and substantially the same descriptions as already provided with respect to FIG. 3 will be omitted below.

The movable support 433 automatically moves the hanger 432. The movable support 433 may have the hanger actuator 434 mounted therein for moving the hanger 432 in a first direction according to an electrical signal transmitted via a terminal 435.

Like in FIG. 3, when the probe rack 430 includes a plurality of hangers 432, the hanger actuators 434 may be respectively provided for the plurality of hangers 432 to independently move the plurality of hangers 432, but embodiment are not limited thereto. As another example, one hanger actuator 434 may move the entire plurality of hangers 432 at the same time.

In detail, the hanger actuator 434 may include one of a stepping motor, a linear motor, etc. For example, the first direction may be a direction of rotation around a connector 432c of the hanger 432. As another example, the first direction may be a vertical direction.

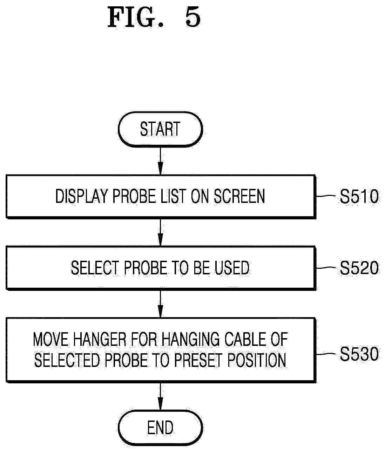

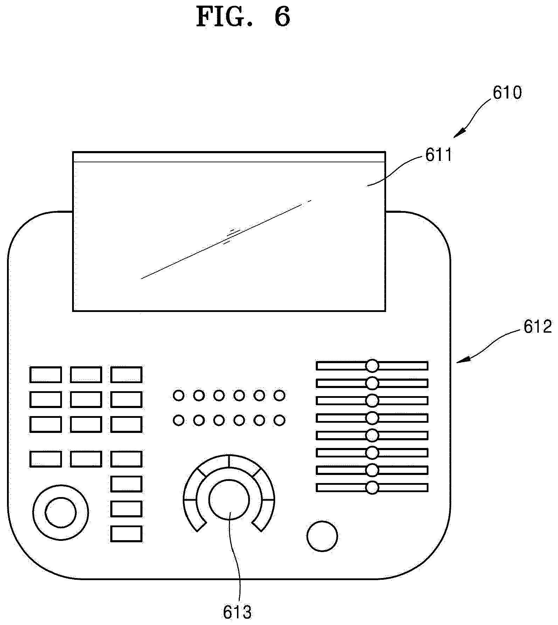

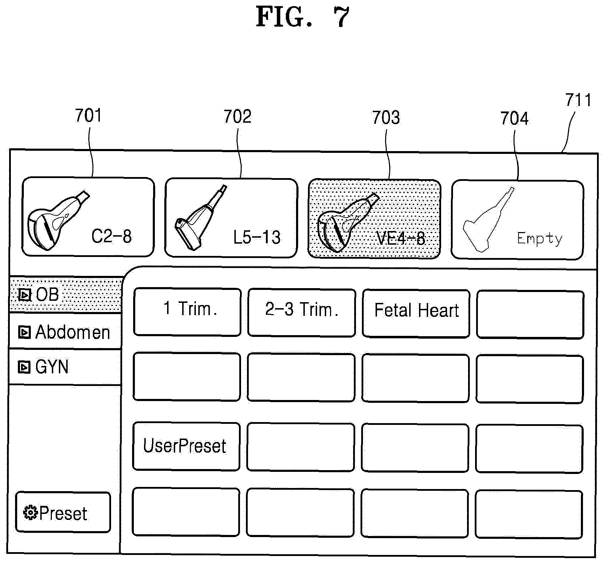

FIG. 5 is a flowchart illustrating an operation of an ultrasound apparatus according to an embodiment, FIG. 6 illustrates a user input interface of an ultrasound apparatus related to an embodiment, and FIG. 7 illustrates an example of a probe setting start screen of an ultrasound apparatus related to an embodiment.

An operation of an ultrasound apparatus employing the probe rack 430 described with reference to FIG. 4 will now be described with reference to FIGS. 5 through 7. The probe rack 430 may be employed in the ultrasound apparatus (100 of FIG. 1 or 200 of FIG. 2).

When the ultrasound apparatus 100 or 200 is in a probe setting mode, a probe list is displayed on a monitor of a touch screen (S510).

According to an embodiment, first through fourth probe lists 701 through 704 are displayed on a probe setting start screen 711, and pieces of probe information are respectively indicated on the first through third probe lists 701 through 703 while information is indicated as 'empty' on the remaining fourth probe list 704. This may mean that the ultrasound apparatus 100 or 200 includes a total of four (4) probe connection terminals, three of which are connected to probes respectively indicated on the first through third probe lists 701 through 703 while the remaining probe connection terminal is not connected to a probe.

In a probe setting mode, soft buttons corresponding to the first through fourth probe lists 701 through 704 are activated. A user selects the probe 140 indicated on the third probe list 703 by selecting the third probe list 703 as shown in FIG. 7 (S520). When the probe setting start screen 711 is displayed on a touch screen 611, the user may select the probe 140 by directly touching the third probe list 703.

The probe setting start screen 711 may be displayed on the display (120 of FIG. 1) instead of a touch screen. When the probe setting start screen 711 is displayed on a general screen such as the display 120, the user may select the probe 140 by clicking on the third probe list 703 via a trackball 613 on a control panel 612 or pressing a hardware button corresponding to the third probe list 703.

According to an embodiment, in a beginning stage of diagnosis, the user may select an object or purpose for such diagnosis. For example, the object or purpose for diagnosis may include cardiac diagnosis, blood vessel diagnosis, abdominal diagnosis, OB/GYN diagnosis, etc. In general, the probes 140 may be categorized into a cardiac probe, a probe for blood vessel and micro-tissue, an abdominal probe, an OB/GYN probe, etc., according to the purpose of use. In the stage of selecting the object or purpose for diagnosis, the ultrasound apparatus100 or 200 may recommend the probe 140 suitable for the selected object or purpose to the user. The hanger actuator 434 may be driven for the hanger 432 corresponding to the recommended probe 140 such that the user may easily select the recommended probe 140. In this case, only when the user selects the recommended probe 140, e. g., by touching the probe 140 on the touch screen 611, the hanger actuator 434 may be driven for the hanger 432 corresponding to the selected probe 140.

When the probe 140 is selected, the controller (270 of FIG. 2) may drive the hanger actuator 434 for moving the hanger 432 over which the cable 142 of the probe 140 corresponding to the selected third probe list 703 is hung. The hanger actuator 434 then moves the hanger 432 to a preset position (S530). For example, the hanger actuator 434 may rotate the hanger 432 such that an end of the hanger 432 is directed toward the probe 140 placed on the probe base 431, thereby allowing the user to easily grip the probe 140. Furthermore, as the hanger 432 moves automatically, the user may easily identify the selected probe 140. Thus, the user may not mistakenly select the wrong probe and is able to hold the probe 140 in his or her hand more quickly.

In addition, when the probe 140 is placed back in the probe rack 430 after use and the cable 142 is hung over the hanger 432, the controller 270 may control the hanger actuator 434 such that the hanger 432 returns to its original position.

Although it has been described that the hanger actuator 434 moves the hanger 432 over which the cable 142 of the selected probe 140 is hung, embodiments are not limited thereto. It would be clearly understood by those of ordinary skill in the art that one hanger actuator 434 may move the entire plurality of hangers 432 simultaneously and in this case, the user may easily hold the probes 140 in his or her hand.

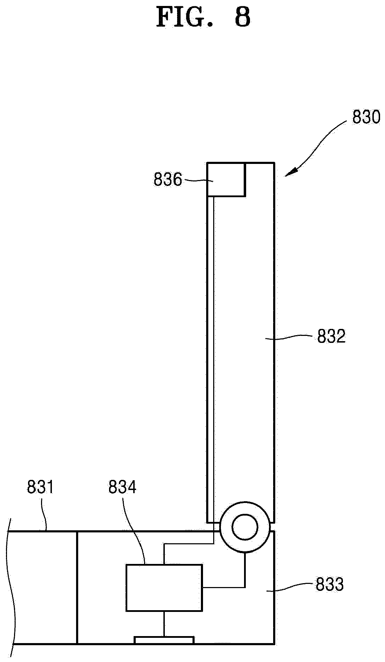

FIG. 8 illustrates a configuration of a probe rack 830 according to an embodiment.

Referring to FIG. 8, the probe rack 830 according to the present embodiment may include a probe base 831, a hanger 832, a movable support 833, a hanger actuator 834, and an indicator light 836. Since the probe rack 830 may have substantially the same configuration as the probe rack 430 described with reference to FIG. 4 except that the probe rack 830 further includes the indicator light 836, only the difference is described, and substantially the same descriptions as already provided with respect to FIG. 4 will be omitted below.

An LED, an OLED, and a lamp may be used as the indicator light 836.

The indicator light 836 may be positioned at one side of the hanger 832. When the probe rack 830 includes a plurality of hangers 832, a plurality of indicator lights 836 may be respectively provided for the plurality of hangers 832.

According to an embodiment, the indicator light 836 may be used to indicate the hanger 832 over which the cable 142 of the probe 140 selected by the user in a probe setting mode is hung. When the indicator light 836 emits light, the user may easily identify the probe 140 to be selected even in a dark place. Thus, the user may not erroneously select the wrong probe and is able to hold the probe 140 in his or her hand more quickly. Furthermore, when the indicator light 836 emits light, it is possible to pay more attention to the hanger 832 that is automatically moved by the hanger actuator 834, thereby reducing the risk of breakage.

Although it has been described that the probe rack 830 of the present embodiment includes both the hanger actuator 834 and the indicator light 836, the probe rack 830 may not include the hanger actuator 834.

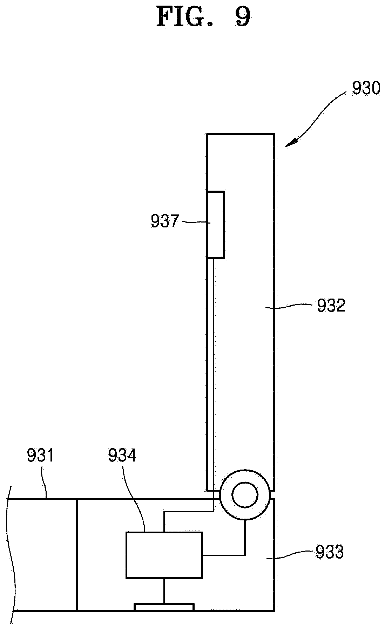

FIG. 9 illustrates a configuration of a probe rack 930 according to an embodiment.

Referring to FIG. 9, the probe rack 930 according to the present embodiment may include a probe base 931, a hanger 932, a movable support 933, a hanger actuator 934, and a cable sensor 937. Since the probe rack 930 may have substantially the same configuration as the probe rack 430 described with reference to FIG. 4 except that the probe rack 930 further includes the cable sensor 937, only the difference is described, and substantially the same descriptions as already provided with respect to FIG. 4 will be omitted below.

The cable sensor 937 may be located on one side of the hanger 932 and include one or a plurality of sensors. The cable sensor 937 detects whether the cable (142 of FIG. 1) of the probe (140 of FIG. 1) is hung over the hanger 932.

According to an embodiment, the cable sensor 937 may be a pressure-sensitive touch sensor for sensing the pressure exerted by the cable 142. As another example, the cable sensor 937 may be a proximity sensor that detects the presence of the cable 142 when the cable 142 approaches the cable sensor 937 within a predetermined distance therefrom without actually touching the cable sensor 937. A proximity sensor refers to a sensor that detects the presence of an object that is approaching or is located near a predetermined detection surface by using the force of an electromagnetic field or infrared light without any mechanical contact. Examples of the proximity sensor include a transmissive photoelectric sensor, a direct reflective photoelectric sensor, a mirror reflective photoelectric sensor, a high-frequency oscillation proximity sensor, a capacitive proximity sensor, a magnetic proximity sensor, and an infrared proximity sensor.

According to an embodiment, the cable sensor 937 senses whether the cable 142 is hung over the hanger 932 and transmits information about whether the cable 142 is hung over the hanger 932 to the controller 270. The controller 270 may control the touch screen (111 of FIG. 1) or the display (120 of FIG. 2) to display a list of the probes 140 hung on the hanger 932, thereby allowing the user to identify a probe that is available for use (. Furthermore, after the probe 140 is used, the cable sensor 937 may detect whether the probe 140 is properly placed back in the probe rack 930. When the probe 140 is not properly held in the probe rack 930, information indicating that the probe 140 is not properly held in the probe rack 930 may be displayed on the touch screen 111 or the display 120, or the indicator light (836 of FIG. 8).may be turned on to indicate such information.

According to an embodiment, the controller 270 may control the touch screen 111 or the display 120 to display information about placement of the probe 140 in the probe rack 930, which is received from the cable sensor 937, together with ID information of the probe 140 checked via the probe connection terminal.

Although it has been described that the probe rack 930 of the present embodiment includes both the hanger actuator 934 and the cable sensor 937, the probe rack 930 may not include the hanger actuator 934. Alternatively, the probe rack 930 may further include the indicator light (836 of FIG. 8) together with the hanger actuator 934 and the cable sensor 937.

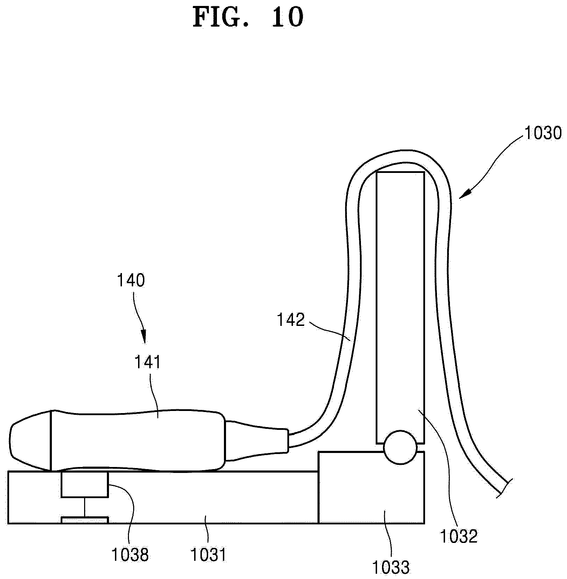

FIG. 10 illustrates a configuration of a probe rack 1030 according to an embodiment.

Referring to FIG. 10, the probe rack 1030 according to the present embodiment may include a probe base 1031, a hanger 1032, a movable support 1033, and a probe sensor 1038. Since the probe rack 1030 may have substantially the same configuration as the probe rack 930 described with reference to FIG. 9 except that the probe rack 1030 includes the probe sensor 1038 instead of the cable sensor 937, only the difference is described, and substantially the same descriptions as already provided with respect to FIG. 9 will be omitted below.

The probe sensor 1038 may be positioned in one side of the probe base 1031 and include one or a plurality of sensors. Sensors in the probe sensor 1038 may be arranged at respective expected positions where probes 140 are to be placed on the probe base 1031. The probe sensor 1038 may sense whether the probe 140 is placed on the probe base 1031.

According to an embodiment, the probe sensor 1038 may be a pressure-sensitive touch sensor for sensing the pressure exerted by the probe 140. As another example, the probe sensor 1038 may be a proximity sensor that detects the presence of the probe 140 when the probe 140 approaches the probe sensor 1038 within a predetermined distance therefrom without actually touching the probe sensor 1038.

According to an embodiment, the probe sensor 1038 senses whether the probe 140 is placed on the probe base 1031 and transmits information about whether the probe 140 is placed on the probe base 1031 to the controller (270 of FIG. 2). The controller 270 may control the touch screen (111 of FIG. 1) or the display (120 of FIG. 2) to display a list of the probes 140 placed on the probe base 1031, thereby allowing the user to identify a probe that is available for use. Furthermore, after the probe 140 is used, the probe sensor 1038 may detect whether the probe 140 is properly placed back in the probe rack 1030. When the probe 140 is not properly held in the probe rack 1030, information indicating that the probe 140 is not properly held in the probe rack 1030 may be displayed on the touch screen 111 or the display 120, or the indicator light (836 of FIG. 8).may be turned on to indicate such information.

The controller 270 may control the touch screen 111 or the display 120 to display information about placement of the probe 140 in the probe rack 1030, which is received from the probe sensor 1038, together with ID information of the probe 140 checked via the probe connection terminal.

Although it has been described that the probe rack 1030 of the present embodiment includes the probe sensor 1038, the probe rack 1030 may further include the cable sensor 937. Furthermore, the probe rack 1030 may further include the hanger actuator (834 of FIG. 8) and the indicator light (836 of FIG. 8).

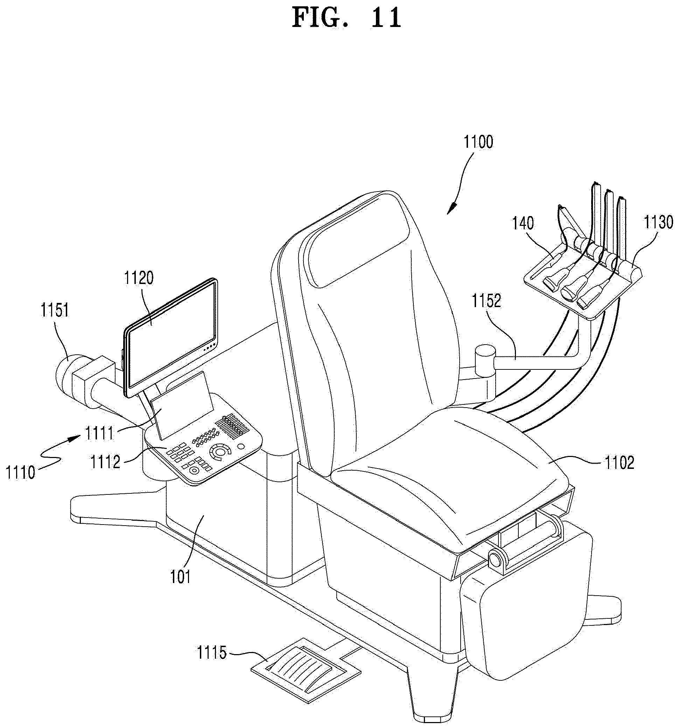

FIG. 11 is an external view of an ultrasound apparatus 1100 according to an embodiment.

Referring to FIG. 11, according to an embodiment, the ultrasound apparatus 1100 may include a main body 1101, a user input interface 1110, a display 1120, and a probe rack 1130. The user input interface 1110, the display 1120, and the probe rack 1130 may each be connected to and supported by the main body 1101. According to an embodiment, the ultrasound apparatus 1100 may further include an examinee chair 1102 on which an examinee is to be seated. The main body 1101 may assume an external appearance that it is separate from the user input interface 1110 and the display 1120, but is not limited thereto as described above.

According to an embodiment, the user input interface 1110 may include a touch screen 1111, a control panel 1112, and a foot switch 1115. The foot switch 1115 may drive a rack actuator (not shown) to move a probe rack support 1152, as will be described below. Since the ultrasound apparatus 1100 according to the present embodiment may have substantially the same configuration as the ultrasound apparatus 100 described with reference to FIG. 1 except that the user input interface 1110 further includes the foot switch 1115, only the difference is described, and substantially the same descriptions as already provided with respect to FIG. 1 will be omitted below.

According to an embodiment, the probe rack 1130 may be spatially separate from the user input interface 1110 (e.g., the control panel 1112). Furthermore, the probe rack 1130 may be separated from the user input interface 1110 such that the main body 1101 or the examinee chair 1102 is located therebetween.

The probe rack 1130 may be movably supported by the probe rack support 1152 extending from the main body 1101. The probe rack support 1152 may be automatically moved by the rack actuator. The rack actuator may be positioned within the probe rack support 1152 or the main body 1101.

The ultrasound apparatus 1100 supports a probe setting mode, and when the probe setting mode is activated, the user may manipulate the foot switch 1115 with his or her foot to move the probe rack 1130.

According to an embodiment, the foot switch 1115 may be a simple on-off switch with a footrest. In this case, in a probe setting mode, the foot switch 1115 in the on-state may move the probe rack 1130 toward the user such that the user may easily hold a probe 140 in his or her hand. The foot switch 1115 may be a switch that enables movement of the probe rack 1130 in one direction, two directions, or four directions (forward, backward, right, and left).

According to an embodiment, the memory (260 of FIG. 2) may store a location of the probe rack 1130 in a probe setting mode such that the probe rack 1130 may automatically move to the stored location when the foot switch 1115 is pressed.

A lever (not shown) may be provided, instead of the foot switch 1115, on the control panel 1112 or other positions that a user's hand may easily reach, and the rack actuator may be driven by manipulating the lever to move the probe rack 1130 toward the user. Also, the rack actuator may be driven via a soft button on the touch screen 1111 or a hardware button on the control panel 1112.

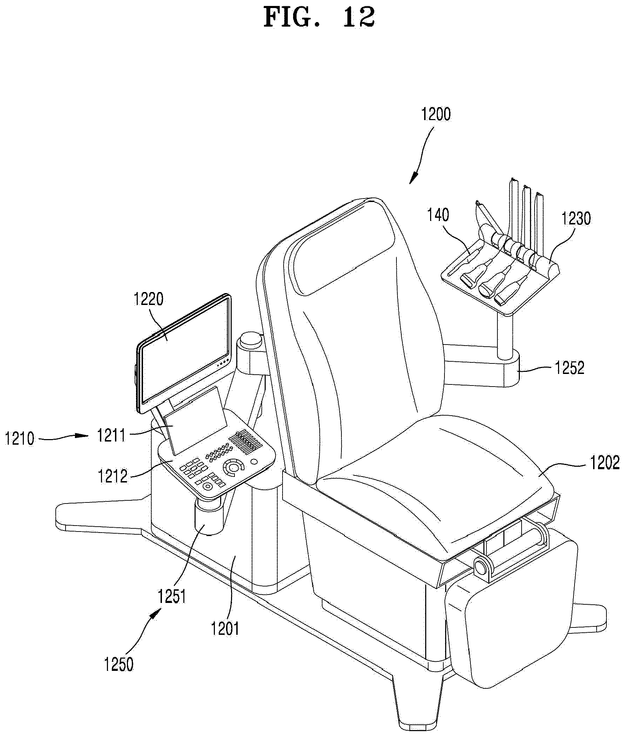

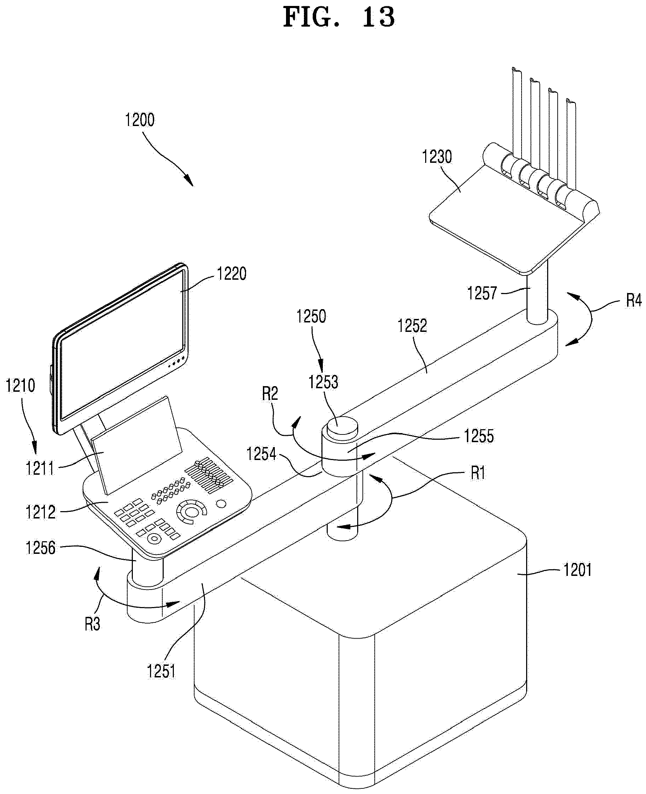

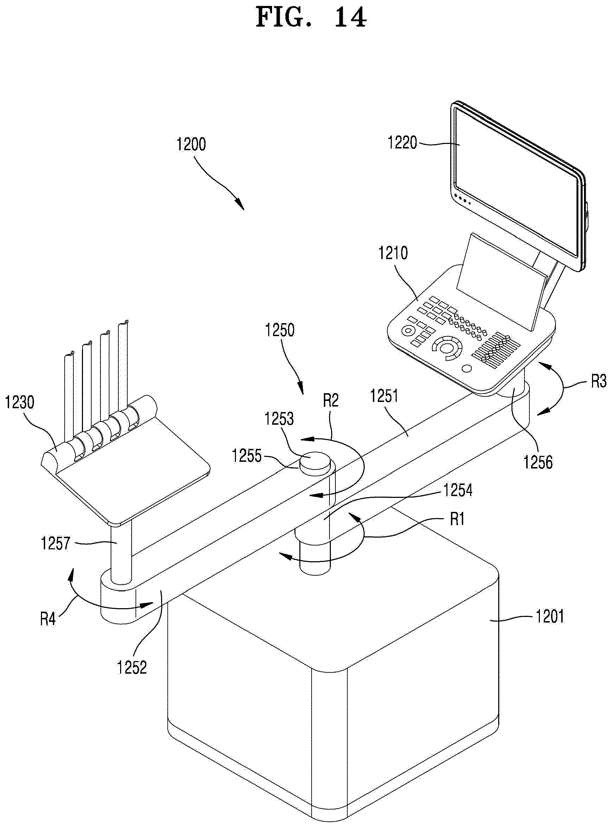

FIG. 12 is an external view of an ultrasound apparatus 1200 according to an embodiment, and FIGS. 13 and 14 are diagrams illustrating operations of the ultrasound apparatus 1200 according to an embodiment. FIGS. 13 and 14 show a state in which an examinee chair 1202 is removed from the ultrasound apparatus 1200 of FIG. 12.

Referring to FIGS. 12 through 14, the ultrasound apparatus 1200 according to the present embodiment may include a main body 1201, the examinee chair 1202, a user input interface 1210, a display 1220, and a probe rack 1230.

The user input interface 1210, the display 1220, and the probe rack 1230 may each be connected to and supported by the main body 1201 by means of a support fixture 1250. Since the ultrasound apparatus 1200 according to the present embodiment may have substantially the same configuration as the ultrasound apparatus 100 described with reference to FIG. 1 except for a structure of the support fixture 1250, only the difference is described, and substantially the same descriptions as already provided with respect to FIG. 1 will be omitted below.

According to an embodiment, the support fixture 1250 may include a first support 1251 for supporting the user input interface 1210 and the display 1220 and a second support 1252 for supporting the probe rack 1230.

The first and second supports 1251 and 1252 are respectively coupled to a support column 1253 provided on the main body 1201 via first and second coupling members 1254 and 1255. The first and second coupling members 1254 and 1255 may be respectively hinges that are capable of independently providing rotations (R1 and R2) around the support column 1253, i.e., a vertical axis. The first and second coupling members 1254 and 1255 are each capable of rotating between 0 degrees and 360 degrees, but are limited thereto. As another example, the maximum allowable angle of rotation of each of the first and second coupling members 1254 and 1255 may be 180 degrees. A hinge coupling structure is an example of a coupling structure for rotatably coupling the first and second supports 1251 and 1252 about the support column 1253, but embodiments are not limited thereto. The support column 1253 may include a lift module configured to lift the first and second coupling members 1254 and 1255 up and down.

The first and second supports 1251 and 1252 may have different lengths with respect to a diameter direction of the support column 1253. The longer one of the first and second supports 1251 and 1252 may be coupled to a lower part of the support column 1253 while the shorter one may be coupled to an upper part thereof.

According to an embodiment, the first coupling member 1254 may be attached to the lower part of the support column 1253, and the second coupling member 1255 may be attached to the upper part thereof. In this case, the first support 1251 may be made longer than the second support 1252 to prevent collision or interference between the user input interface 1210 and the probe rack 1230 during rotation of the first and second supports 1251 and 1252. As another example, the first and second coupling members 1254 and 1255 may be respectively attached to the upper and lower parts of the support column 1253, and the first support 1251 may be shorter than the second support 1252.

The first support 1251 may be movably coupled to a control panel 1212 via a third coupling member 1256. The third coupling member 1256 is capable of providing rotation R3 of the control panel 1212 about a vertical axis, but is not limited thereto. As another example, the third coupling member 1256 may include a lift module capable of providing up/down lift as well as rotation (R3) movement. As another example, the third coupling member 1256 may be capable of providing tilt movement such that a tilt of the control panel 1212 may be adjusted.

The display 1220 may be attached to the control panel 1212 or may be supportably coupled to the first support 1251 together with the control panel 1212. The display 1220 may also be coupled to the control panel 1212 or the first support 1251 via a hinge or other various coupling members capable of providing various movements, such that the display 1220 may move relative to the control panel 1212.

The second support 1252 movably supports the probe rack 1230 via a fourth coupling member 1257. The fourth coupling member 1257 is capable of providing rotation R4 of the probe rack 1230 about a vertical axis, but is not limited thereto. As another example, the fourth coupling member 1257 may include a lift module capable of providing up/ down lift as well as rotation (R4) movement. As another example, the fourth coupling member 1257 may be capable of providing tilt movement such that a tilt of the probe rack 1230 may be adjusted.

The first or second support 1251 or 1252 may rotate around the support column 1253 such that the user input interface 1210 or the probe rack 1230 may move toward or away from the user.

Furthermore, as shown in FIG. 13, when the first support 1251 located on the left side of the main body 1201 rotates 180 degrees clockwise or counterclockwise, the first support 1251 may be located on the right side of the main body 1201 as shown in FIG. 14. Accordingly, the user input interface 1210 may move from left to right with respect to the main body 1201.

Similarly, as shown in FIG. 13, when the second support 1252 located on the right side of the main body 1201 rotates 180 degrees clockwise or counterclockwise, the second support 1252 may be located on the ;left side of the main body 1201 as shown in FIG. 14. Accordingly, the probe rack 1230 may move from right to left with respect to the main body 1201.

Some users may be familiar with the way of manipulating the user input interface 1210 with the left hand while performing a test with the probe 140 held in the right hand. On the other hand, other users may be familiar with the way of manipulating the user input interface 1210 with the right hand while performing a test with the probe 140 held in the left hand. The ultrasound apparatus 1200 according to the present embodiment is capable of changing the locations of the user input interface 1210 and the probe rack 1230 as described above, thereby accommodating both left- and right-handed users.

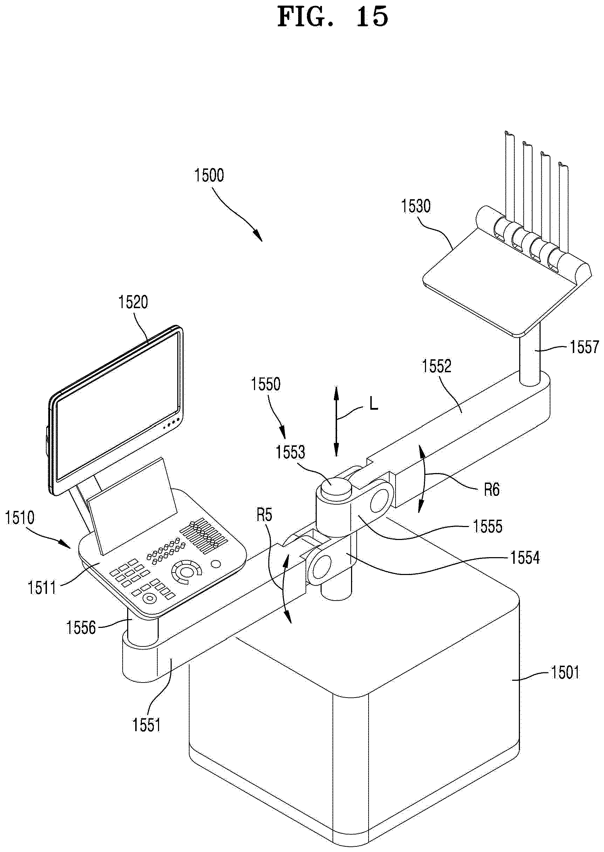

FIG. 15 is a diagram illustrating operation of an ultrasound apparatus 1500 according to an embodiment.

Referring to FIG. 15, the ultrasound apparatus 1500 according to the present embodiment may include a main body 1501, a user input interface 1510, a display 1520, and a probe rack 1530. The user input interface 1510, the display 1520, and the probe rack 1530 may each be connected to and supported by the main body 1501 by means of a support fixture 1550. Since the ultrasound apparatus 1500 according to the present embodiment may have substantially the same configuration as the ultrasound apparatus 1200 described with reference to FIGS. 12 through 14 except for a structure of the support fixture 1550, only the difference is described, and substantially the same descriptions as already provided with respect to FIGS. 12 through 14 will be omitted below.

According to an embodiment, the support fixture 1550 may include a first support 1551 for supporting the user input interface 1510 and the display 1520 and a second support 1552 for supporting the probe rack 1530.

The first and second supports 1551 and 1552 are respectively coupled to a support column 1553 provided on the main body 1501 via first and second coupling members 1554 and 1555. The first and second coupling members 1554 and 1555 may each be a double hinge with a hinge, which is capable of rotation about a vertical axis, being integrated with a hinge, which is capable of rotation about a horizontal axis.

The first and second coupling members 1554 and 1555 may be coupled to the support column 1553 such that they may independently rotate about the support column 1553. The first and second coupling members 1554 and 1555 may each rotate about the support column 1553, i.e., a vertical axis, through 360 degrees, but are not limited thereto. Furthermore, the first and second coupling members 1554 and 1555 may be capable of respectively providing rotations R5 and R6 of the first and second supports 1551 and 1552 about a horizontal axis. The heights of the user input interface 1510 and the probe 1530 may be respectively adjusted due to the rotations R5 and R6 of the first and second coupling members 1554 and 1555 about the horizontal axis.

The first support 1551 may be movably coupled to a control panel 1511 via a third coupling member 1556. The third coupling member 1556 may couple the control panel 1511 to the first support 1551 to enable not only rotation of the control panel 1511 about a vertical axis but also tilt movement such that a tilt of the control panel 1511 may be adjusted. When the height of the control panel 1511 of the user input interface 1510 is adjusted by the first support 1551, the level of the control panel 1511 may be maintained by adjusting the tilt of the control panel 1511 via the third coupling member 1556.

The second support 1552 may be movably coupled to the probe rack 1530 via a fourth coupling member 1557. The fourth coupling member 1557 may couple the probe rack 1530 to the second support 1552 to enable not only rotation of the probe rack 1530 about a vertical axis but also tilt movement such that a tilt of the probe rack 1530 may be adjusted. When the height of the probe rack 1530 is adjusted by the second support 1552, the level of the probe rack 1530 may be maintained by adjusting the tilt of the probe rack 1530 via the fourth coupling member 1557.

A structure of the first and second coupling members 1554 and 1555 according to the present embodiment is an example of a coupling structure that enables rotation of the user input interface 1510 and the probe rack 1530 about a vertical axis and their height adjustment, but embodiments are not limited thereto.

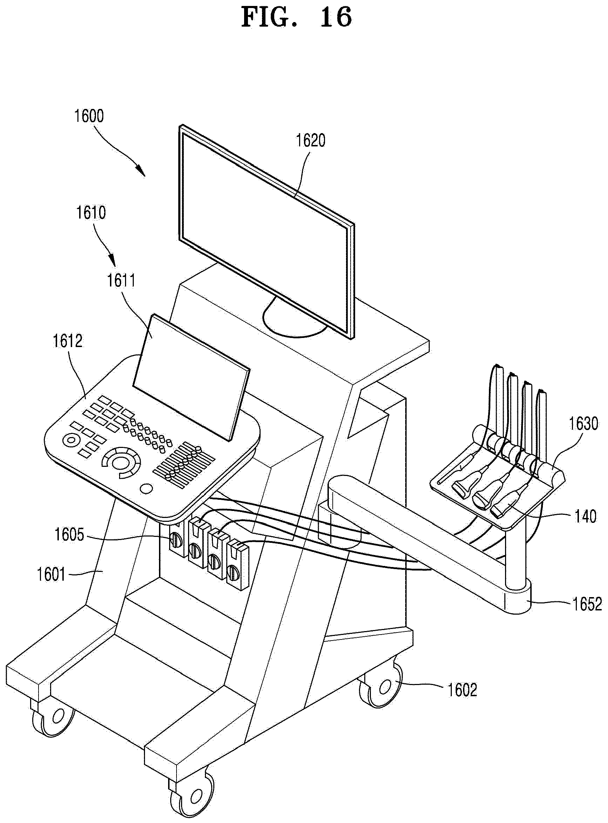

FIG. 16 is an external view of an ultrasound apparatus 1600 according to an embodiment.

Referring to FIG. 16, the ultrasound apparatus 1600 according to the embodiment may include a main body 1601, a user input interface 1610, a display 1620, and a probe rack 1630. Casters 1602 are provided beneath the main body 1601 to facilitate movement of the ultrasound apparatus 1600. A probe connection terminal 1605 is provided on one side of the main body 1601 and connects a probe 140 to the main body 1601. Since the ultrasound apparatus 1600 according to the present embodiment may have substantially the same configuration as the ultrasound apparatus 100 described with reference to FIG. 1 except that the ultrasound apparatus 1600 is a mobile cart-type apparatus, only the difference is described, and substantially the same descriptions as already provided with respect to FIG. 1 will be omitted below.

According to an embodiment, the user input interface 1610 may be connected to the front of the main body 1601. Although not shown in FIG. 16, a control panel 1612 of the user input interface 1610 may be coupled to the main body 1601 to enable tilt movement, forward-backward movement, left-right rotation, or a combination of these movements. A touch screen 1611 may be fixedly or tiltably attached to the control panel 1612. Alternatively, the touch screen 1611 may be coupled to the main body 1601 via a separate support member. The display 1620 is mounted to a top of the main body 1601, but is not limited thereto.

The main body 1601 may assume an external appearance that it is separate from the user input interface 1610 and the display 1620, but is not limited thereto. For example, the main body 1601 may be externally integrated with the control panel 1612 of the user input interface 1610 or the display 1620.

The probe rack 1630 is spatially separated from the user input interface 1610.

According to an embodiment, the probe rack 1630 may be supported by a probe rack support 1652 extending from a side of the main body 1602. The probe rack support 1652 may enable movement of the probe rack 1630 via a coupling structure having at least one degree of freedom. For example, the probe rack support 1652 may be coupled to the probe rack 1630 to enable the probe rack 1630 to rotate about a vertical axis or move up or down.

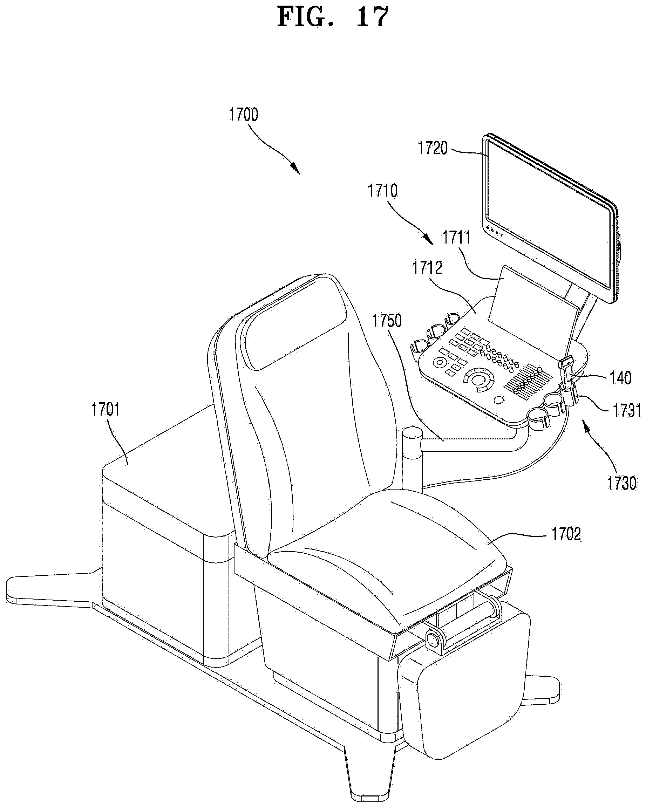

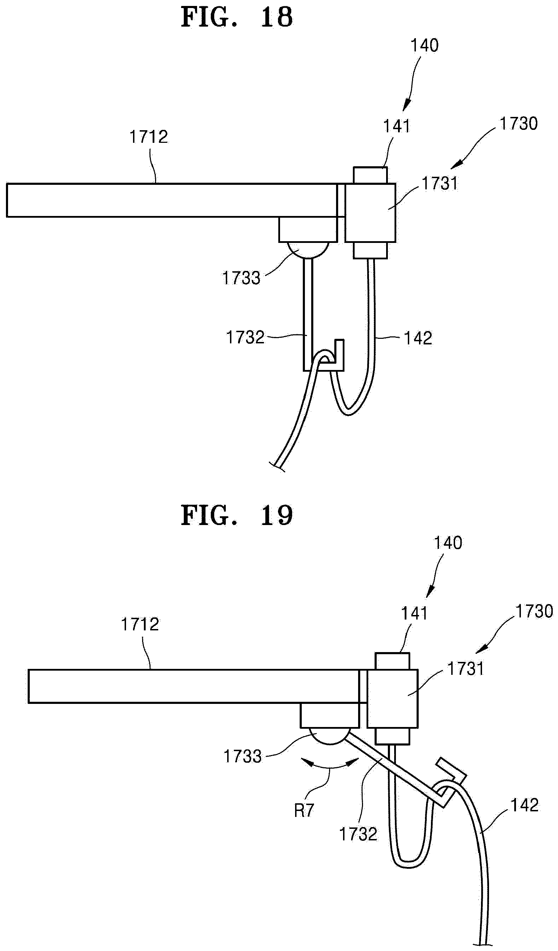

FIG. 17 is an external view of an ultrasound apparatus 1700 according to an embodiment, and FIGS. 18 and 19 are diagrams illustrating a probe rack 1730 according to an embodiment.

Referring to FIGS. 17 through 19, the ultrasound apparatus 1700 according to the embodiment may include a main body 1701, a user input interface 1710, a display 1720, and a probe rack 1730. According to an embodiment, the ultrasound apparatus 1700 may further include an examinee chair 1702 on which an examinee is to be seated. Since the ultrasound apparatus 1700 according to the present embodiment may have substantially the same configuration as the ultrasound apparatus 100 described with reference to FIG. 1 except for a configuration and a location of the probe rack 1730, only the difference is described, and substantially the same descriptions as already provided with respect to FIG. 1 will be omitted below.

The user input interface 1710 and the display 1720 may each be connected to and supported by the main body 1701. The main body 1701 may assume an external appearance that it is separate from the user input interface 1710 and the display 1720, but is not limited thereto. For example, the main body 1701 may be externally integrated with the user input interface 1710 or the display 1720.

According to an embodiment, the user input interface 1710 may include a touch screen 1711 and a control panel 1712. The control panel 1712 may be movably coupled to the main body 1701 via a support 1750.

The probe rack 1730 may include a probe base 1731, a hanger 1732, and a movable support 1733.

The probe base 1731 is located at one side of the control panel 1712, but is not limited thereto. As another example, the probe rack 1730 may be located on a side surface of the main body 1701 or the examinee chair 1702. According to an embodiment, the probe base 1731 may have a form of a holder into which a probe body 141 is fit, but is not limited thereto. As another example, the probe base 1731 may have a form of a shelf.

According to an embodiment, the hanger 1732 may be located below the control panel 1712. The probe rack 1730 may include a plurality of hangers 1732, but embodiments are not limited thereto.

The hanger 1732 includes a hanging section over which a cable 142 of a probe 140 is hung. For example, the hanger 1732 may have a hooked hanging section.

The movable support 1733 couples the hanger 1732 to the control panel 1712 and supports the hanger 1732 to enable it to move manually or automatically. The movable support 1733 may be formed integrally with or be detachably attached to the control panel 1712.

According to an embodiment, as shown in FIGS. 18 and 19, the movable support 1733 may couple the hanger 1732 to the control panel 1712 to enable rotation R7 of the hanger 1732 about a horizontal rotation axis, but embodiments are not limited thereto. As another example, the movable support 1733 may extend or stretch out the hanger 1732 in a horizontal direction. As another example, the movable support 1733 may rotate the hanger 1732 while extending/stretching out the same.

The probe body 141 of the probe 140 is placed in the probe base 1731, and the cable 142 is draped down by its own weight. In this case, the hanger 1732 is lowered downwardly, and the cable 142 is hung over the hanger 1732. When the user holds the probe 140 in his or her hand and pulls it, the hanger 1732 rotates such that its end faces upwards, and the cable 142 moves upward together with the hanger 1732 and then falls out of the hanger 1732 such that the probe 140 is ready for use. In the ultrasound apparatus 1700 according to the present embodiment, the hanger 1732 is configured to hang the cable 142, thereby dispersing the weight of the cable 142. Furthermore, as the hanger 1732 moves, the cable 142 naturally comes out of the hanger 1732 when the user pulls the probe 140 for use. Thus, in the ultrasound apparatus 100 according to the embodiment, the user may feel less weight of the cable 142 when pulling the probe 140 for use, and thus user convenience may be improved. Furthermore, even when the plurality of probes 140 are respectively connected to probe connection terminals (not shown) of the ultrasound apparatus 1700, it is possible to prevent their corresponding cables 142 from being entangled with one another by hanging parts of the cables 142 over the hanger 1732.

According to an embodiment, an elastic member, a shock absorber, a damper, or the like may be added to the hanger 1732 such that the hanger 1732 may support the weight of the cable 142 or move more smoothly.

According to an embodiment, the movable support 1733 may further include a hanger actuator (not shown) configured to automatically move the hanger 1732 in response to an electrical signal. In this case, when the probe 140 is selected on the user input interface 1710, the hanger actuator may automatically move the hanger 1732 over which the cable 142 of the probe 140 is hung. As another example, when the probe 140 is selected on the user input interface 1710, the hanger actuator may automatically move all the hangers 1732.

According to an embodiment, a probe rack and an ultrasound apparatus employing the same are configured to hang a heavy cable of a probe over a hanger and enable the hanger to move, thereby providing improved user convenience.

According to an embodiment, the probe rack and the ultrasound apparatus employing the same may provide manipulation convenience for both left- and right-handed users.

A method according to an embodiment may be implemented in the form of program instructions that may be performed by various types of computers and may be recorded on non-transitory computer-readable media. The non-transitory computer-readable media may include program instructions, data files, data structures, etc. either alone or in combination. The program instructions recorded on the non-transitory computer-readable media the medium may be designed and configured specially for the present disclosure or may be known to and usable by those skilled in the art of computer software. Examples of non-transitory computer-readable media include magnetic media such as hard disks, floppy disks, and magnetic tape, optical media such as compact disk-ROM (CD-ROM) and digital versatile disks (DVDs), magneto-optical media such as floptical disks, and hardware devices that are specially configured to store and perform program instructions, such as ROM, RAM, flash memory, and the like. Examples of program instructions include not only machine code such as that created by a compiler but also higher level code that may be executed by a computer using an interpreter or the like.

While one or more embodiments have been described with reference to the figures, it will be understood by those of ordinary skill in the art that the embodiments are not to be construed as limiting the scope of the present disclosure. Various changes and modifications in form and details based on the basic concept of the disclosure may fall within the spirit and scope as defined by the following claims.

## Claims

1. A probe rack comprising:
a probe base on which a probe is placed;
a hanger over which a probe cable of the probe is hung; and
a movable support configured to movably support the hanger with respect to the probe base.

2. The probe rack of claim 1, wherein the hanger is located at one side of the probe base, and
optionally, wherein the hanger extends in one direction.

3. The probe rack of claim 1 or 2, wherein the hanger is located below the probe base.

4. The probe rack of any one of the preceding claims, wherein the movable support is formed integrally with or is detachably attached to the probe base.

5. The probe rack of any one of the preceding claims, further comprising a hanger actuator that is controlled by an electrical signal to move the hanger in a first direction with respect to the probe base.

6. The probe rack of claim 5, wherein the hanger comprises first and second hangers, and
wherein the hanger actuator is configured to independently move the first and second hangers.

7. The probe rack of any one of the preceding claims, further comprising a probe sensor configured to detect whether the probe is placed on the probe base, and optionally an indicator light configured to emit light according to a result of the detecting by the probe sensor.

8. The probe rack of any one of the preceding claims, further comprising a cable sensor configured to detect whether the probe cable is hung over the hanger, and optionally an indicator light configured to emit light according to a result of the detecting by the cable sensor.

9. An ultrasound apparatus comprising:
a probe configured to transmit and receive ultrasound signals; and
a probe rack according to any one of the preceding claims, which is configured to hold the probe.

10. An ultrasound apparatus of claim 9, the ultrasound apparatus comprising:
a main body; and
a user input interface configured to input a control command according to manipulation by a user,
wherein the probe rack is spatially separated from the user input interface such that the main body is interposed between the probe rack and the user input interface.

11. The ultrasound apparatus of claim 10, further comprising an examinee chair or an examinee table located in a space for accommodating an examinee,
wherein the probe rack is spatially separated from the user input interface such that the examinee chair or the examinee table is interposed between the probe rack and the user input interface.

12. The ultrasound apparatus of claim 10 or 11, further comprising a probe rack support configured to support the probe rack while enabling the probe rack to move independently of the main body.

13. The ultrasound apparatus of claim 12, wherein the probe rack support is further configured to support the probe rack while enabling the probe rack to move from one side of the main body to an opposite side thereof.

14. The ultrasound apparatus of any of claims 10-13, further comprising a user input interface support configured to movably support the user input interface, wherein the user input interface support is further configured to support the user input interface while enabling the user input interface to move from one side of the main body to an opposite side thereof.

15. The ultrasound apparatus of any of claims 9-14, further comprising a control panel configured to input a control command according to manipulation by a user,
wherein the probe base is attached to a side surface of the control panel, and the hanger is located below the control panel.
